# EUROPÄISCHE PATENTSCHRIFT

(11) **EP 0 618 768 B1**
(45) Veröffentlichungstag und Bekanntmachung des Hinweises auf die Patenterteilung: **20.11.1996**
(21) Anmeldenummer: 92924720.3
(22) Anmeldetag: 11.12.1992
(51) Int. Cl.: A01N 59/00

(54) **WUNDANTISEPTIKUM**
ANTISEPTIC
ANTISEPTIQUE POUR PLAIES ET BLESSURES

(30) Priorität: 20.12.1991 DE 4142319
(43) Veröffentlichungstag der Anmeldung: 12.10.1994
(73) Patentinhaber: Henkel Kommanditgesellschaft auf Aktien, 40191 Düsseldorf (DE)
(72) Erfinder: FRIESE, Carsten, D-2000 Hamburg 56 (DE); MAINZ, Beate, D-3550 Marburg (DE); DISCH, Karl-Heinz, D-5657 Haan (DE); HACHMANN, Klaus, D-4010 Hilden (DE); BANSEMIR, Klaus-Peter, D-4018 Langenfeld (DE); KRÄCHTER, Hans-Udo, D-4630 Bochum (DE)
(86) Internationale Anmeldenummer: EP9202876
(87) Internationale Veröffentlichungsnummer: WO9312657

(56) Entgegenhaltungen:
- EP-A- 0 016 319
- EP-A- 0 231 080
- EP-A- 0 252 278
- EP-A- 0 383 005
- EP-A- 0 411 315
- WO-A-91/02538
- DE-A- 2 811 756
- FR-A- 1 539 262
- FR-A- 1 583 994
- FR-A- 2 356 600
- GB-A- 1 539 771
- US-A- 2 451 149
- US-A- 5 043 484
- PATENT ABSTRACTS OF JAPAN vol. 13, no. 224 (C-599)(3572) 24. Mai 1989

## Beschreibung

Die intakte Haut, die eine natürliche Schutzfunktion ausübt und den menschlichen Körper weitgehend vor Infektionen durch Mikroorganismen schützt, braucht in der Regel nicht desinfiziert zu werden. Eine Ausnahme gilt selbstverständlich für Ärzte, Pflegepersonal, etc.. Bei einer durch eine Wunde geschädigten Haut sollte diese jedoch nach einer gründlichen Reinigung stets desinfiziert werden, um ein Eindringen von Infektionserregern in das Wundgebiet und das umliegende Gewebe oder gar in die Blutbahn zu verhindern.

Als Wunde wird jede durch äußere Einwirkung wie Gewalt, Hitze, Kälte, Chemikalien oder Strahlen hervorgerufene gewaltsame Durchtrennung oder Zerstörung der Haut, der Schleimhaut, der tieferen Gewebe oder der inneren Organe bezeichnet. Je nach ihrer Entstehungsursache kann man eine Vielzahl von Wunden unterscheiden, die hinsichtlich ihrer äußeren Merkmale recht unterschiedlich ausgeprägt sein und dementsprechend unterschiedliche Infektionsgefährdungen zeigen können.

Unmittelbar nach einer Verletzung setzen im Organismus komplexe zelluläre und biochemische Prozesse ein, mit dem Ziel, den erlittenen Gewebedefekt zu reparieren. Diese Wundheilungsprozesse laufen bei stabilem Allgemeinzustand des Patienten spontan ab. Voraussetzung für eine sich problemlos vollziehende Wundheilung ist jedoch eine sorgfältige Erstversorgung nach offener Wunden, denn die Heilung einer Wunde wird am stärksten durch eine Wundinfektion gestört. Alle anderen Störfaktoren sind nachgeordneter Natur. Vordringliches Ziel jeder Wundbehandlung muß deshalb sein, eine Wunde keimfrei, bzw. keimarm zu halten. Nach der erfolgten Erstversorgung einer frischen Wunde durch gründliche Reinigung und einem eventuellen chirurgischen Eingriff steht ein breites Spektrum an Präparaten zur Wunddesinfektion zur Verfügung.

Marktführend bei der desinfizierenden Wundbehandlung sind die sogenannten PVP-Jod-Präparate, die Polyvinylpyrrolidon-Jod-Komplexe mit 10 % verfügbarem Jod enthalten.

Jod wird seit ca. 150 Jahren in Form einer meist ethanolischen Jodtinktur oder wäßrigen Lösung (beide mit Zusatz von KJ) zur Wunddesinfektion verwendet. Über die Verträglichkeit von Jodlösungen sind viele Untersuchungen durchgeführt worden. Sie zeigen, daß Jod zwar zu den wirksamsten Antiseptika auf dem Gebiet gehört, daneben aber auch einige Nachteile aufweist. So wird Jod durch die Haut aufgenommen. Das führt zu Erhöhung des Serumjodspiegels und kann systemische Nebenwirkungen nach sich ziehen. In ca. 1 % der Anwendungsfälle treten allergische Reaktionen auf. Bei ungeklärter Schilddrüsenfunktion kann es zu einer jodinduzierten Hyperthyreose kommen. Ein weiterer entscheidender Nachteil ist, daß die verfügbare Menge an Jod in diesen Jodtinkturen kaum konstant gehalten werden kann. Wegen dieser Schwierigkeiten ist man seit langem dazu übergegangen, die sog. Jodophore zur Wunddesinfektion einzusetzen. Jodophore sind Komplexe von hochmolekularen, oberflächenaktiven Trägerstoffen mit Jod. Am gebräuchlichsten ist hier der eingangs erwähnte Polyvinylpyrrolidon-Jod-Komplex (PVP-Jod) mit 10 % verfügbarem Jod. Das Wirkungsspektrum entspricht im wesentlichen dem des elementaren Jods. Die Humanverträglichkeit von PVP-Jod ist aber ebenfalls umstritten und wird in der Literatur immer wieder kontrovers diskutiert. Da auch hier Jod durch die Haut resorbiert wird, entsprechen die Nebenwirkungen der PVP-Jod-Komplexe den Nebenwirkungen des reinen Jods.

Da aber gerade bei Wunden die Gefahr der Jodresorption und der damit verbundenen Nebenwirkungen besonders groß ist, bestand die Notwendigkeit für die Entwicklung eines jodfreien Wundantiseptikums. Bei der Entwicklung eines solchen Wundantiseptikums war besonders darauf zu achten, daß toxikologisch und physiologisch unbedenkliche Wirkstoffe eingesetzt werden, da nicht ausgeschlossen werden kann, daß diese durch verletzte Blutgefäße in den Blutkreislauf gelangen und eine systemische Wirkung auf den gesamten Körper ausüben können.

Unter diesen Gesichtspunkten erschien Wasserstoffperoxid als Basiswirkstoff geeignet für die Entwicklung eines neuen jodfreien Wundantiseptikums.

Die Anwendung von wasserstoffperoxidhaltigen Zusammensetzungen für dermatologische bzw. desinfizierende Zwecke ist z.B. aus der deutschen Offenlegungsschrift 32 05 318, der deutschen Patentschrift 23 27 181, der britischen Patentschrift 1 539 771 und der Chemiker-Zeitung, 99 (1975), Seiten 132 - 137, bekannt. Wasserstoffperoxid ist eine farblose, wasserklare, fast geruchlose, schwach sauer reagierende Flüssigkeit, die mit Wasser in jedem Verhältnis mischbar ist. Desgleichen ist es in einer Vielzahl von organischen Lösungsmitteln sowie pharmazeutischen Hilfsstoffen gut löslich. Wasserstoffperoxid besitzt ein breites Wirkungsspektrum gegen Bakterien, Pilze und Viren. Je nach Konzentration und Einwirkzeit wirkt es mikrobistatisch oder mikrobizid.

Im deutschen Arzneibuch, 9. Ausgabe (DAB9) wird Wasserstoffperoxid als 30 %- und 3 %ige Lösung aufgeführt. In der Medizin dient die konzentrierte Lösung (30 %) nur zur Herstellung verdünnter Wasserstoffperoxid-Lösungen. Da Wasserstoffperoxid leicht zersetzt werden kann, können die Lösungen einen geeigneten Stabilisator enthalten.

Die Stabilität wäßriger Wasserstoffperoxid-Lösungen ist sehr vom pH-Wert abhängig. Bei niedriger Konzentration können oft inaktivierende Komponenten nicht ausreichend kompensiert werden, während ab ≧ 3 % H₂O₂-Gehalt Störfaktoren weitgehend ausgeschaltet werden. Zur Stabilisierung wäßriger Wasserstoffperoxid-Lösungen gegen katalytisch wirkende Metallionen werden überwiegend Komplexbildner, wie etwa 8-Hydroxy-chinolin, aber auch niedere, aliphatische Alkohole (Ethanol, n-Propanol, iso-Propanol) eingesetzt.

Sowohl 8-Hydroxy-chinolin als auch die vorstehend zitierten aliphatischen und auch aromatische Alkohole wie z.B. Phenoxyethanol und verschiedene ihrer Derivate haben bekanntlich teilweise selbst desinfizierende Wirkung. Auch eine Reihe von quaternären Ammoniumverbindungen haben als Desinfektionsmittel Bedeutung erlangt, beispielsweise Didecyldimethylammoniumchlorid, Benzethoniumchlorid oder Cetylpyridiniumchlorid.

Auch Bis-guanidin-derivate wie Chlorhexidin werden seit Jahren als anitmikrobielle Wirkstoffe verwendet.

Alle diese Verbindungen können zwar zusammen mit Wasserstoffperoxid eingesetzt werden, sie stabilisieren dieses aber nicht immer und steigern dessen Wirkung nur teilweise.

Es wurde nun gefunden, daß der Zusatz von Phenoxyethanol zu Wasserstoffperoxid zu einem besonders wirksamen und gewebeverträglichen Wundantiseptikum führt. Die Konzentration des Mittels an Wasserstoffperoxid soll dabei 0,05 bis 3, vorzugsweise 1,5 bis 3, insbesondere etwa 2 bis 3 Gew.-% betragen. Die Konzentration an Phenoxyethanol soll dabei ebenfalls 0,05 bis 3, vorzugsweise 1,5 bis 3, insbesondere etwa 2 bis 3 Gew.-%.

Zusätze von 5 bis 15, vorzugsweise 7,5 bis 10 Gew.-% an stabilisierende Alkoholen wie Ethanol und vorzugsweise Isopropanol hatten keinen negativen Einfluß auf die mikrobiologische Wirksamkeit. Auch ein Zusatz von 0,01 bis 0,1 Gew.-%, vorzugsweise von 0,025 bis 0,04 Gew.-% an 8-Hydroxy-chinolin wirkt als Stabilisator für Wasserstoffperoxid, entfaltet aber in dieser Konzentration keine besondere eigene antimikrobielle Wirksamkeit.

Aus toxikologischer und physiologischer Sicht ist der Einsatz von Isopropanol generell und auch dem anderer aliphatischer Alkohole vorzuziehen. Ebenfalls aus physiologischen Gründen werden als Lösungsmittel die sogenannten Ringer-, Ringer-Lactat- oder physiologische Kochsalzlösung, jeweils nach DAB9 eingesetzt, die in ihrer Zusammensetzung in etwa der der Körperflüssigkeiten entsprechen und deshalb keinen zusätzlichen Reiz auf die Wunde ausüben.

### Beispiele

Alle im folgenden beschriebenen Ansätze wurden im 1500 g Ansatz durchgeführt. Alle in den Rezepturen vorkommenden Prozentangaben bedeuten Gewichtsprozent. Die hergestellten Lösungen wurden in PE- oder Glasflaschen gelagert. Die Lagerung bei Raumtemperatur erfolgte im Labor, die Lagerung bei 40 °C in einem Trockenschrank der Firma Heraeus und die bei 0 °C in einer Klimakammer.

Zum Nachweis der antimikrobiellen Wirksamkeit eines Wundantiseptikums wurden mit einer festgelegten Wirkstoffmenge getränkte sterilisierte Mullkompressen auf Keimagarplatten mit definierter Keimzahl (Testkeime hier: Staphylococcus aureus, Pseudomonas aeruginosa) aufgelegt. Die geschlossenen Petrischalen wurden bei 37 °C für die Dauer der Einwirkzeit inkubiert. Nach Ablauf einer Einwirkzeit von 2 Stunden, 4 Stunden und 24 Stunden wurden die Mullkompressen entfernt. Aus der vorher mit Mull bedeckten Zone des Keimagars wurde mit Hilfe eines Korkbohrers ein Agarstanzling entnommen. Dieser wurde in eine Inaktivierungslösung aufgenommen, homogenisiert und die Keimzahl bestimmt. Zur Kontrolle wurde immer ein Wasserwert (Mullkompresse mit Wasser getränkt) angesetzt, der bei der Auswertung der Ergebnisse als Bezugspunkt für die Ermittlung des Reduktionsfaktors herangezogen wurde.

Zur Überprüfung des Wirkstoffes unter Blut- und Eiweißbelastung wurde dem Keimagar bzw. der Desinfektionsmittellösung entweder 5 % Blut oder 0,2 % Rinderserumalbumin zugesetzt. Als Referenzsubstanz wurde standardisierte unverdünnte PVP-Jod-Lösung (Handelspräparat Betaisodona, Mundipharma, 10 % verfügbares Jod) getestet.

Als optimal ergab sich die Wirkstoffkombination:
2,0 % Wasserstoffperoxid
2,0 % Phenoxyethanol
10,0 % Isopropanol,
in wäßrig-physiologischen Lösungen, die mikrobiologisch gut wirksam ist.

| Testkeim | Reduktionsfaktor nach | |
|---|---|---|
| | 2 Std. | 24 Std. |
| Staphylococcus aureus | 1,63 | 3,14 |

Bei der Referenzsubstanz betrug der Reduktionsfaktor nach 2 Stunden 0,42 und nach 24 Stunden 3,68.

Die Rezeptur war in dieser Form problemlos zu konfektionieren (s.u.). Sie wurde zusätzlich unter Blutbelastung mit den Keimen Staphylococcus aureus und Pseudomonas aeruginosa getestet. Die Ergebnisse (Reduktionsfaktoren) dieser Teste waren sehr gut.

| Testkeim | Reduktionsfaktor nach | | |
|---|---|---|---|
| | 2 Std. | 4 Std. | 24 Std. |
| Staphylococcus aureus | 3,5 | 2,75 | >5,33 |
| Pseudomonas aeruginosa | 4,26 | >5,7 | >5,7 |

Die Referenzsubstanz (Betaisadona, Mundipharma) zeigte folgende antimikrobielle Wirksamkeiten:

| Testkeim | Reduktionsfaktor nach | | |
|---|---|---|---|
| | 2 Std. | 4 Std. | 24 Std. |
| Staphylococcus aureus | >5,29 | 4,8 | >5,29 |
| Pseudomonas aeruginosa | >5,7 | >5,7 | >5,7 |

Zur Überprüfung der Lagerstabilität wurde der Wasserstoffperoxid-Gehalt einiger ausgewählter Lösungen regelmäßig festgestellt. Der Wasserstoffperoxid-Gehalt wurde mit einem Titroprozessor 686 der Fa. Methrom (TFC-OC, H. Küster) durch jodometrische Titration bestimmt. Zur Vorbereitung der Probe wird eine definierte Einwaage der zu prüfenden Lösung mit je 20 ml Isopropanol und destilliertem Wasser versetzt, mit 10 ml 10 %iger Schwefelsäure angesäuert und mit 1 ml 3 %iger Molybdatlösung versetzt. Nach Zugabe einer Spatelspitze Kaliumjodid wird sofort mit einer 0,1 n Natriumthiosulfat-Lösung titriert.

Der Wasserstoffperoxidgehalt dieser Rezepturen war über 100 Tage bei 40 °C stabil:

| | H₂O₂-Gehalt (%) |
|---|---|
| T(0) Ansatztag | 2,17 |
| T(1) nach 100 d | 2,08 |

Dieses Ergebnis konnte auch photometrisch bestätigt werden. Die Stabilität von Phenoxyethanol wurde durch chromotographische Bestimmung nachgewiesen.

Die vorstehend angegebenen Bestimmungen erfolgten auch noch mit jeweils 0,1 % Thymol, Benzethoniumchlorid, Didecyldimethylammoniumchlorid, Cetylpyridiniumchlorid, Hexetidin und Chlorhexidin sowie 0,05 und 0,1 % 8-Hydroxychinolin anstelle von Phenoxyethanol. Es wurden jedoch entweder schlechtere mikrobiolische Ergebnisse erzielt, die Verbindungen erwiesen sich als teilweise cytotoxisch, oder die Lösung war nicht lagerstabil.

## Patentansprüche

1. Wäßrig-physiologische wasserstoffperoxidhaltige Lösungen mit einem Gehalt an 0,05 bis 3,0 Gew.-% Phenoxyethanol und 0,05 bis 3,0 Gew.-% an Wasserstoffperoxid.

2. Wäßrig-physiologische Lösungen nach Anspruch 1 mit einem weiteren Gehalt an 8-Hydroxy-chinolin.

3. Wäßrig-physiologische Lösungen nach Anspruch 1 und 2 mit einem Gehalt an 0,01 bis 0,1 Gew.-% an 8-Hydroxy-chinolin.

4. Wäßrig-physiologische Lösungen nach Anspruch 1 bis 3 mit einem zusätzlichen Gehalt an Stabilisatoren für Wasserstoffperoxid.

5. Wäßrig-physiologische Lösungen nach Anspruch 1 bis 4 mit einem Zusatz an Ethanol, n-Propanol und insbesondere Isopropanol als Stabilisator für Wasserstoffperoxid.

6. Wäßrig-physiologische Lösungen nach Anspruch 1 bis 5 mit einem Gehalt an 0,1 bis 2 Gew.-% Wasserstoffperoxid als Wundantiseptikum.

7. Wäßrig-physiologische Lösungen nach Anspruch 1 bis 6 mit einem Gehalt von 0,1 bis 2 Gew.-% Phenoxyethanol.

8. Wäßrig-physiologische Lösungen nach Anspruch 1 bis 7 mit einem Zusatz von 5 bis 15, vorzugsweise 7,5 bis 10 Gew.-% Ethanol, n-Propanol und insbesondere Isopropanol.

## Claims

1. Aqueous physiological hydrogen peroxide solutions containing 0.05 to 3.0% by weight of phenoxyethanol and 0.05 to 3.0% by weight of hydrogen peroxide.

2. Aqueous physiological solutions as claimed in claim 1 additionally containing 8-hydroxyquinoline.

3. Aqueous physiological solutions as claimed in claims 1 and 2 containing 0.01 to 0.1% by weight of 8-hydroxyquinoline.

4. Aqueous physiological solutions as claimed in claims 1 to 3 additionally containing stabilizers for hydrogen peroxide.

5. Aqueous physiological solutions as claimed in claims 1 to 4 containing an addition of ethanol, n-propanol and, more particularly, isopropanol as a stabilizer for hydrogen peroxide.

6. Aqueous physiological solutions as claimed in claims 1 to 5 containing 0.1 to 2% by weight of hydrogen peroxide as a wound antiseptic.

7. Aqueous physiological solutions as claimed in claims 1 to 6 containing 0.1 to 2% by weight of phenoxyethanol.

8. Aqueous physiological solutions as claimed in claims 1 to 7 containing an addition of 5 to 15% by weight and preferably 7.5 to 10% by weight of ethanol, n-propanol and, more particularly, isopropanol.

## Revendications

1. Solutions aqueuses-physiologiques d'eau oxygénée, renfermant 0,05 à 3,0 % en poids de phénoxyéthanol et 0,05 à 3,0 % en poids d'eau oxygénée.

2. Solutions aqueuses-physiologiques selon la revendication 1, renfermant en outre de la 8-hydroxyquinoléine.

3. Solutions aqueuses-physiologiques selon les revendications 1 et 2, renfermant 0,01 à 0,1 % en poids de 8-hydroxyquinoléine.

4. Solutions aqueuses-physiologiques selon les revendications 1 à 3, additionnées de stabilisants pour l'eau oxygénée.

5. Solutions aqueuses-physiologiques selon les revendications 1 à 4, additionnées d'éthanol, de n-propanol et, en particulier, d'isopropanol, comme stabilisant pour l'eau oxygénée.

6. Solutions aqueuses physiologiques selon les revendications 1 à 5, renfermant 0,1 à 2 % en poids d'eau oxygénée, comme antiseptique pour les plaies.

7. Solutions aqueuses-physiologiques selon les revendications 1 à 6, renfermant 0,1 à 2 % en poids de phénoxyéthanol.

8. Solutions aqueuses-physiologiques selon les revendications 1 à 7, additionnées de 5 à 15, de préférence, de 7,5 à 10 % en poids d'éthanol, de n-propanol et, en particulier, d'isopropanol.
